# FASCICULE DE BREVET EUROPEEN

(11) **EP 1 877 560 B1**
(45) Date de publication et mention de la délivrance du brevet: **25.02.2015**
(21) Numéro de dépôt: 06764593.7
(22) Date de dépôt: 03.05.2006
(51) Int. Cl.: C12N 15/56, C12N 9/24, A23K 1/165

(54) **GENE ABFB-2 DE PENICILLIUM FUNICULOSUM**
ABFB-2 GEN AUS PENICILLIUM FUNICULOSUM
ABFB-2 GENE OF PENICILLIUM FUNICULOSUM

(30) Priorité: 04.05.2005 FR 0504560
(43) Date de publication de la demande: 16.01.2008
(73) Titulaire: Adisseo France S.A.S., 92160 Antony (FR)
(72) Inventeur: FRANCOIS, Jean Marie, F-31320 Castenet-Tolosan (FR); PARROU, Jean-Luc, F-31400 Toulouse (FR); TOURRASSE, Olivier Résidence les gémeaux, F-31100 Toulouse (FR); NORE, Olivier, F-37210 Vernou sur Brenne (FR)
(74) Mandataire: Jouannic, Nathalie
(86) Numéro de dépôt international: PCT/FR2006/000997
(87) Numéro de publication internationale: WO 2006/117481

(56) Documents cités:
- WO-A-99/57325
- WO-A-2004/018662
- CARVALLO MARCELA ET AL: "Characterization of an alpha-L-arabinofuranosidase gene (abf1) from Penicillium purpurogenum and its expression." MYCOLOGICAL RESEARCH, vol. 107, no. 4, avril 2003 (2003-04), pages 388-394, XP009059573 ISSN: 0953-7562 cité dans la demande
- DE IOANNES PABLO ET AL: "An alpha-L-arabinofuranosidase from Penicillium purpurogenum: Production, purification and properties" JOURNAL OF BIOTECHNOLOGY, vol. 76, no. 2-3, 21 janvier 2000 (2000-01-21), pages 253-258, XP002395553 ISSN: 0168-1656
- PARK NYUN HO ET AL: "A new method for the preparation of crystalline L-arabinose from arabinoxylan by enzymatic hydrolysis and selective fermentation with yeast" BIOTECHNOLOGY LETTERS, vol. 23, no. 5, mars 2001 (2001-03), pages 411-416, XP002361552 ISSN: 0141-5492
- DATABASE EMBL 14 novembre 2001 (2001-11-14), HASHIMOTO T ET AL: "Alpha-L-arabinofuranosidase of Aspergillus oryzae HL15" XP002395261 Database accession no. AB073860
- DATABASE UniProt 1 décembre 2001 (2001-12-01), HASHIMOTO T ET AL: "Alpha-L-arabinofuranosidase B" XP002395262 Database accession no. Q96VA1
- CLINCHE LE F ET AL: "ALPHA-L-ARABINOFURANOSIDASES FROM ASPERGILLUS TERREUS WITH POTENTIAL APPLICATION IN ENOLOGY: INDUCTION, PURIFICATION, AND CHARACTERIZATION" JOURNAL OF AGRICULTURAL AND FOOD CHEMISTRY, AMERICAN CHEMICAL SOCIETY. WASHINGTON, US, vol. 45, no. 7, juillet 1997 (1997-07), pages 2379-2383, XP000656899 ISSN: 0021-8561
- MARGOLLES-CLARK E ET AL: "CLONING OF GENES ENCODING ALPHA-L-ARABINOFURANOSIDASE AND BETA-XYLOSIDASE FROM TRICHODERMA REESEI BY EXPRESSION IN SACCHAROMYCES CEREVISIAE" APPLIED AND ENVIRONMENTAL MICROBIOLOGY, WASHINGTON,DC, US, vol. 62, no. 10, octobre 1996 (1996-10), pages 3840-3846, XP000900963 ISSN: 0099-2240

### LISTE DE SEQUENCES

<110> ADISSEO FRANCE SAS
<120> Gène *abfB*-2 de *Penicillium funiculosum*
<130> KH/SG/BR049664
<140>
   <141>
<160> 9
<170> PatentIn Ver. 2.1
<210> 1
   <211> 1639
   <212> ADN
   <213> Pénicillium funiculosum
<220>
   <221> promoter
   <222> (1) .. (267)
<220>
   <221> TATA_signal
   <222> (167)..(172)
<220>
   <221> CDS
   <222> (268)..(1470)
<220>
   <221> sig_peptide
   <222> (268) .. (348)
<220>
   <221> misc_binding
   <222> (1366)..(1467)
   <223> fCBD cellulose binding domain
<220>
   <221> misc_feature
   <222> (1285)..(1353)
   <223> Domain of low complexity
<220>
   <221> terminator
   <222> (1471)..(1639)
<220>
   <221> polyA_signal
   <222> (1620)..(1625)
<400> 1
   ctagtgattg atcaagcacc atacccctct tccattctgg gataatatga cggcatatac 60
   gttacatcag catacaatat agtcaaatta tcggttaaaa ggatgtattt gaattatttg 120
   aattatccga ttttaaagac tccttcatag tgtgcgttag aagcagtata aagcccgctt 180
   caatagctag cagttgaatc gatacccact cgaacaacat ttttgaaacg caattgatac 240
   ttgtatagtc tcacaaaacg attcaac atg acg tcc aaa cat agt ttc gaa cga 294
                                 Met Thr Ser Lys His Ser Phe Glu Arg
                                  1               5
   gcc ggc ata ctt gca ttg ggc ctt att gct acg agc tct ctt gtt gcc   342
   Ala Gly Ile Leu Ala Leu Gly Leu Ile Ala Thr Ser Ser Leu Val Ala
    10                  15                  20                  25
   gcc ggc cct tgt gac atc tac tct tca ggt ggc aca cca tgc gtt gcc   390
   Ala Gly Pro Cys Asp Ile Tyr Ser Ser Gly Gly Thr Pro Cys Val Ala
                   30                  35                  40
   gcg cac agt acc act cgc gca ctc tat gat gct tat act ggc ccg cta   438
   Ala His Ser Thr Thr Arg Ala Leu Tyr Asp Ala Tyr Thr Gly Pro Leu
               45                  50                  55
   tac caa gtg aca cgg agt tct gat agc agc aag aaa gat atc gcg cca   486
   Tyr Gln Val Thr Arg Ser Ser Asp Ser Ser Lys Lys Asp Ile Ala Pro
           60                  65                  70
   ttg gcc gcc ggc ggc gtt gct aat gct gcc acg caa gac tcc ttt tgt   534
   Leu Ala Ala Gly Gly Val Ala Asn Ala Ala Thr Gln Asp Ser Phe Cys
       75                  80                  85
   tca gga aca acc tgc ctc ata tct atc atc tac gac caa tct gga aag   582
   Ser Gly Thr Thr Cys Leu Ile Ser Ile Ile Tyr Asp Gln Ser Gly Lys
    90                  95                 100                 105
   ggg aac cat ctc acc caa gct ccg aaa ggt ggc tgg agt gga cct gga   630
   Gly Asn His Leu Thr Gln Ala Pro Lys Gly Gly Trp Ser Gly Pro Gly
                   110                 115                 120
   cca aat ggt tca gat aat tta tcc agt gcg acc gcc gcc cca atc tat   678
   Pro Asn Gly Ser Asp Asn Leu Ser Ser Ala Thr Ala Ala Pro Ile Tyr
               125                 130                 135
   ctg aac gga caa aag gcg tac ggc gtg ttt att gca cct ggt gac ggc   726
   Leu Asn Gly Gln Lys Ala Tyr Gly Val Phe Ile Ala Pro Gly Asp Gly
           140                 145                 150
   tac cgt aat gat aag act tct ggt ata gcc aca ggc gat caa ccc gag   774
   Tyr Arg Asn Asp Lys Thr Ser Gly Ile Ala Thr Gly Asp Gln Pro Glu
       155                 160                 165
   gga atg tac gcc atc ttt gac gga acg cac tac aac ggc ggc tgc tgc   822
   Gly Met Tyr Ala Ile Phe Asp Gly Thr His Tyr Asn Gly Gly Cys Cys
   170                 175                 180                 185
   ttc gac tac ggt aat gct gaa acc agt ggt acc gat aca ggc gct ggc   870
   Phe Asp Tyr Gly Asn Ala Glu Thr Ser Gly Thr Asp Thr Gly Ala Gly
                   190                 195                 200
   cac atg gag gct atc tac ttc ggt aac tgt aat gtc tgg ggt tct ggt   918
   His Met Glu Ala Ile Tyr Phe Gly Asn Cys Asn Val Trp Gly Ser Gly
               205                 210                 215
   tct gga tca ggc cct tgg att atg gct gat ttg gag aat ggt ctc ttc   966
   Ser Gly Ser Gly Pro Trp Ile Met Ala Asp Leu Glu Asn Gly Leu Phe
           220                 225                 230
   tcc ggt tat aac gcc aaa caa aac acc gcc gat gca tcc atc aac tgg   1014
   Ser Gly Tyr Asn Ala Lys Gln Asn Thr Ala Asp Ala Ser Ile Asn Trp
       235                 240                 245
   cga ttc gtc act gca att gtg aag ggc gag cca aac aat tgg gca atc    1062
   Arg Phe Val Thr Ala Ile Val Lys Gly Glu Pro Asn Asn Trp Ala Ile
   250                 255                 260                 265
   cgt ggt ggc aat gcc caa tct ggt tct ctc tcg aca tac tat aat ggc    1110
   Arg Gly Gly Asn Ala Gln Ser Gly Ser Leu Ser Thr Tyr Tyr Asn Gly
                   270                 275                 280
   ata cgc cca tca ggc tac aat ccg atg cac aaa gaa ggc gcc att atc    1158
   Ile Arg Pro Ser Gly Tyr Asn Pro Met His Lys Glu Gly Ala Ile Ile
               285                 290                 295
   ctc ggc acg ggt ggt gac aac agt aac ggt gct caa ggc act ttt tac    1206
   Leu Gly Thr Gly Gly Asp Asn Ser Asn Gly Ala Gln Gly Thr Phe Tyr
           300                 305                 310
   gag ggt gtg atg act tct ggg tac cct tct gac tca act gag aat tcc    1254
   Glu Gly Val Met Thr Ser Gly Tyr Pro Ser Asp Ser Thr Glu Asn Ser
       315                 320                 325
   gtt caa gcc aat atc gtt gcc gcc ggt tat tcc act tcg cct ggt agc    1302
   Val Gln Ala Asn Ile Val Ala Ala Gly Tyr Ser Thr Ser Pro Gly Ser
   330                 335                 340                 345
   cac acc act tcc acc acc ctt acc acc atc act agt acc aca gca gta    1350
   His Thr Thr Ser Thr Thr Leu Thr Thr Ile Thr Ser Thr Thr Ala Val
                   350                 355                 360
   tct gga gct ggc cag aca cac tgg ggt cag tgt gga ggt agc gga tac    1398
   Ser Gly Ala Gly Gln Thr His Trp Gly Gln Cys Gly Gly Ser Gly Tyr
               365                 370                 375
   tcc ggt cca acg agc tgt gtt gca ccc tac gct tgt aca acc gct aac    1446
   Ser Gly Pro Thr Ser Cys Val Ala Pro Tyr Ala Cys Thr Thr Ala Asn
           380                 385                 390
   cct tac tac gct caa tgt ctc tag aatataggcg ctcattcgtt ctatgactga   1500
   Pro Tyr Tyr Ala Gln Cys Leu
       395                 400
   agttggacaa gtatcaaaag ctctctggag gcagggagtg tatttttgat gattataccg 1560
   tctggagaaa gtgtatatag cttctcataa cccagacaat cagatatttc taacagagca 1620
   ataaatgaga gatgatcaa                                               1639
<210> 2
   <211> 400
   <212> PRT
   <213> Pénicillium funiculosum
<400> 2
   Met Thr Ser Lys His Ser Phe Glu Arg Ala Gly Ile Leu Ala Leu Gly
    1               5                  10                  15
   Leu Ile Ala Thr Ser Ser Leu Val Ala Ala Gly Pro Cys Asp Ile Tyr
               20                  25                  30
   Ser Ser Gly Gly Thr Pro Cys Val Ala Ala His Ser Thr Thr Arg Ala
           35                  40                  45
   Leu Tyr Asp Ala Tyr Thr Gly Pro Leu Tyr Gln Val Thr Arg Ser Ser
       50                  55                  60
   Asp Ser Ser Lys Lys Asp Ile Ala Pro Leu Ala Ala Gly Gly Val Ala
   65                  70                  75                  80
   Asn Ala Ala Thr Gln Asp Ser Phe Cys Ser Gly Thr Thr Cys Leu Ile
                   85                  90                  95
   Ser Ile Ile Tyr Asp Gln Ser Gly Lys Gly Asn His Leu Thr Gln Ala
               100                 105                 110
   Pro Lys Gly Gly Trp Ser Gly Pro Gly Pro Asn Gly Ser Asp Asn Leu
           115                 120                 125
   Ser Ser Ala Thr Ala Ala Pro Ile Tyr Leu Asn Gly Gln Lys Ala Tyr
       130                 135                 140
   Gly Val Phe Ile Ala Pro Gly Asp Gly Tyr Arg Asn Asp Lys Thr Ser
   145                 150                 155                 160
   Gly Ile Ala Thr Gly Asp Gln Pro Glu Gly Met Tyr Ala Ile Phe Asp
                   165                 170                 175
   Gly Thr His Tyr Asn Gly Gly Cys Cys Phe Asp Tyr Gly Asn Ala Glu
               180                 185                 190
   Thr Ser Gly Thr Asp Thr Gly Ala Gly His Met Glu Ala Ile Tyr Phe
           195                 200                 205
   Gly Asn Cys Asn Val Trp Gly Ser Gly Ser Gly Ser Gly Pro Trp Ile
       210                 215                 220
   Met Ala Asp Leu Glu Asn Gly Leu Phe Ser Gly Tyr Asn Ala Lys Gln
   225                 230                 235                 240
   Asn Thr Ala Asp Ala Ser Ile Asn Trp Arg Phe Val Thr Ala Ile Val
                   245                 250                 255
   Lys Gly Glu Pro Asn Asn Trp Ala Ile Arg Gly Gly Asn Ala Gln Ser
               260                 265                 270
   Gly Ser Leu Ser Thr Tyr Tyr Asn Gly Ile Arg Pro Ser Gly Tyr Asn
           275                 280                 285
   Pro Met His Lys Glu Gly Ala Ile Ile Leu Gly Thr Gly Gly Asp Asn
       290                 295                 300
   Ser Asn Gly Ala Gln Gly Thr Phe Tyr Glu Gly Val Met Thr Ser Gly
   305                 310                 315                 320
   Tyr Pro Ser Asp Ser Thr Glu Asn Ser Val Gln Ala Asn Ile Val Ala
                   325                 330                 335
   Ala Gly Tyr Ser Thr Ser Pro Gly Ser His Thr Thr Ser Thr Thr Leu
               340                 345                 350
   Thr Thr Ile Thr Ser Thr Thr Ala Val Ser Gly Ala Gly Gln Thr His
           355                 360                 365
   Trp Gly Gln Cys Gly Gly Ser Gly Tyr Ser Gly Pro Thr Ser Cys Val
       370                 375                 380
   Ala Pro Tyr Ala Cys Thr Thr Ala Asn Pro Tyr Tyr Ala Gln Cys Leu
   385                 390                 395                 400
<210> 3
   <211> 34
   <212> PRT
   <213> Pénicillium funiculosum
<400> 3
   Thr His Trp Gly Gln Cys Gly Gly Ser Gly Tyr Ser Gly Pro Thr Ser
   1               5                   10                  15
   Cys Val Ala Pro Tyr Ala Cys Thr Thr Ala Asn Pro Tyr Tyr Ala Gln
               20                  25                  30
   Cys Leu
<210> 4
   <211> 23
   <212> PRT
   <213> Pénicillium funiculosum
<400> 4
   Ser Thr Ser Pro Gly Ser His Thr Thr Ser Thr Thr Leu Thr Thr Ile
   1               5                   10                  15
   Thr Ser Thr Thr Ala Val Ser
               20
<210> 5
   <211> 34
   <212> PRT
   <213> Pénicillium funiculosum
<400> 5
   Ala His Trp Ala Gln Cys Gly Gly Ile Gly Tyr Ser Gly Cys Thr Ala
   1               5                   10                  15
   Cys Ala Ser Pro Tyr Thr Cys Gln Lys Ala Asn Asp Tyr Tyr Ser Gln
               20                  25                  30
   Cys Leu
<210> 6
   <211> 34
   <212> PRT
   <213> Pénicillium funiculosum
<400> 6
   Ala His Trp Gly Gln Cys Gly Gly Ile Gly Trp Ser Gly Pro Thr Ile
   1               5                   10                  15
   Cys Val Ser Pro Tyr Thr Cys Gln Val Leu Asn Pro Tyr Tyr Ser Gln
               20                  25                  30
   Cys Leu
<210> 7
   <211> 34
   <212> PRT
   <213> Pénicillium funiculosum
<400> 7
   Ala His Trp Gly Gln Cys Gly Gly Gln Gly Trp Thr Gly Pro Thr Thr
    1               5                  10                  15
   Cys Ala Ser Gly Thr Thr Cys Thr Val Val Asn Pro Tyr Tyr Ser Gln
               20                  25                  30
   Cys Leu
<210> 8
   <211> 25
   <212> ADN
   <213> Séquence artificielle
<220>
   <223> Description de la séquence artificielle: Amorce XbaI-abfB-2
<400> 8
   tctagaatga cgtccaaaca tagtt 25
<210> 9
   <211> 24
   <212> ADN
   <213> Séquence artificielle
<220>
   <223> Description de la séquence artificielle: Amorce HindIII-abfB-2
<400> 9
   aagcttctag agacattgag cgta 24

## Description

L'invention concerne le gène *abfB-2* isolé de *Penicillium funiculosum* et le polypeptide ABFB-2 codé par ce gène ayant une activité α-L-arabinofuranosidase B.

*Penicillium funiculosum* est un Talaromyces appartenant à la famille des *Aspergilleae.* L'isolement de ce microorganisme à partir de nombreux substrats organiques sujets à une contamination aérienne ou aqueuse, indique que ce champignon possède une panoplie d'enzymes hydrolytiques d'une richesse surprenante. L'utilisation de ce cocktail enzymatique dans l'alimentation animale contribue à la dépolymérisation des substances organiques naturelles et permet d'améliorer leur digestibilité. WO 99/57325 décrit ainsi une souche de *Penicillium funiculosum* dénommée IMI378536 produisant un mélange d'enzymes particulièrement adapté à l'alimentation animale. Néanmoins les cocktails enzymatiques produits par *Penicillium funiculosum* sont peu caractérisés biochimiquement. En effet seul un nombre restreint d'activités enzymatiques comme les xylanases, les β-glucanases sont en général mesurées sur les moûts de fermentation obtenus. Ces activités ne reflètent qu'une fraction de la population enzymatique présente dans le cocktail.

Les composés hémi-cellulolytiques issus de l'agriculture constituent la deuxième réserve de polysaccharides après la cellulose au sein des tissus végétaux. Ce groupe est caractérisé par une large variété d'hétéropolysaccharides, dont les principaux représentants sont les xylans, les arabinanes, les galactanes, les glucanes, et les mannanes. L'arabinose sous sa forme furfural est largement représenté au sein des hétéropolysaccharides tels que les arabinanes et les arabinoxylanes. L'arabinane est un polymère de résidus arabinofuranose liés par des liaisons α-1-5 et il peut être substitué par 1 ou 2 résidus arabinose en position O-2 ou O-3. En ce qui concerne les arabinoxylanes, les résidus α-L-arabinofuranosyl sont liés sur la chaîne principale β-1-4-xylopyranosyl par liaisons α-1-3 et α-1-2. La présence de résidus arabinose sur ces chaînes latérales peut restreindre l'hydrolyse enzymatique des composés hémi-cellulolytiques dans de nombreuses applications industrielles telles que l'amélioration de la digestibilité de l'alimentation animale. Les enzymes clivant les liaisons α-L-arabinofuranosidiques peuvent agir synergiquement avec les xylanases pour permettre l'hydrolyse des arabinoxylanes et arabinanes.

Les activités arabinases (endo-, exo-arabinases et majoritairement les activités ∝-L-arabinofuranosidases) peuvent donc contribuer activement et de façon synergique avec les xylanases à la dépolymérisation des composés hemi-cellulolytiques. Les composés hemi-cellulolytiques et pectiques peuvent représenter jusqu'à 50% des carbohydrates totaux présents dans les plantes et ils constituent une importante source d'énergie pour les animaux. L'amélioration de la digestibilité de ces composés est corrélée avec la diminution du degré de substitution des résidus arabinosyls au sein des composés hémi-cellulolytiques (Brice, R.E., Morrison, I.M. 1982, Carbohydr. Res. 101 : 93-100).

Les enzymes hydrolysant les liaisons entre des résidus L-arabinose ont été isolées à partir de micro-organismes tels que les bactéries ou les champignons filamenteux. Les arabinosidases sont constituées principalement d'α-L-arabinofuranosidases (EC 3.2.1.55) qui sont capables d'hydrolyser les résidus α-L-arabinofuranosyl non réducteurs issus du L-arabinoxylane ou de composés tels que les arabinanes, et les arabinogalactanes.

Les α-L-arabinofuranosidases (EC 3.2.1.55) ont été classées en deux familles de Glycosides Hydrolases (GH 51 et GH 54) selon leur similarités de séquence protéique. Ces deux familles diffèrent de par leur spécificité de substrat contenu dans les polysaccharides. Le premier groupe (GH 51) contient les arabinofuranosidases de type A qui agissent seulement sur de petites structures linéaires d'arabinofuranosyl oligosaccharides liés en α-1-5. Le second groupe est constitué d'arabinofuranosidases de type B (GH 54) qui catalysent l'hydrolyse des liaisons α-1,5 ; α-1,3 et α-1,2 des chaînes latérales contenues dans les composés arabinofuranosyl-oligosaccharides.

Les arabinofuranosidases B (ABFB) ont été isolées à partir de nombreuses bactéries mais également à partir de champignons filamenteux. Le genre *Aspergillus* est le plus représenté, mais elles ont été également isolées à partir des genres *Trichoderma, Penicillium, et Fusarium.*

WO96/29416, WO96/06935 et US 5,989,887 décrivent des gènes d'arabinofuranosidase *d'Aspergillus niger.*

Gielkens et al. (Microbiology, 145, 735-741, 1999) ont décrit le gène *abfB d'Aspergillus nidulans.*

WO96/29416, WO96/06935, WO 2004/018662 et US 5,989,887 décrivent des gènes d'arabinofuranosidase d'*Aspergillus niger.* L'alignement de séquences protéiques indique que la protéine abfB de *A. niger* est à 50,9% identique à la protéine ABFB-2 de *P. funiculosum.* Aucune des caractéristiques essentielles à l'utilisation du polypeptide en nutrition animale n'est décrite dans ces demandes.

Clinche et al. (J. Agric. Food Chem., 45, 2379-2383, 1997) ont décrit trois α-L-arabinofuranosidases issues de *Aspergillus terreus* ayant une application potentielle en oenologie.

Les gènes *abfB* d'*Aspergillus kawachii* et *d'Aspergillus awamori* ont été décrits par Koseki et al. (J. of Bioscience and Bioengineering, vol. 96, no. 3, 232-241, 2003). Ces enzymes ont des applications dans la fermentation de la liqueur japonaise *shochu.*

Le gène *abfB* du champignon filamenteux *Trichoderma reesei* a été décrit par Margolles-Clark et al. (Applied and Environmental Microbiology, 3840-3846, 1996).

Panagiotou *et al* ont également décrits deux alpha-L-arabinofuranosidases extracellulaires issues de *Fusarium oxysporum.* (Can J Microbiol. 2003 : 49(10):639-4).

Carvallo et al. (Mycol. Res., 107 (4), 388-394, 2003) ont décrit l'α-L-arabinofuranosidase B de *Penicillium purpurogenum.* L'alignement de séquences protéiques indique que la protéine abf-1 de *P. purpurogenum* est à 51,2% identique à la protéine ABFB-2 de *P. funiculosum.* Aucune des caractéristiques essentielles à l'utilisation du polypeptide en nutrition animale n'est décrite dans cet article.

Sakamoto et al. (FEBS Letters 560, 199-204, 2004) ont décrit le gène *abnx* de *Penicillium chrysogenum* codant cependant pour une activité arabinanase distincte de l'activité des ABFB.

Cependant, ces enzymes ABFB n'ont pas les qualités optimales requises pour une application en alimentation animale.

En effet, pour être utilisables en alimentation animale, les ABFB doivent posséder des propriétés compatibles avec les traitements que subissent les aliments destinés à cette alimentation. En particulier, l'activité des enzymes utilisées doit être stable dans les conditions de température et de pH des procédés, et si possible être optimale dans la préparation de ces aliments ainsi que dans les conditions présentes dans le système digestif des animaux ingérant ces aliments.

De plus, ces enzymes doivent avoir un large spectre d'action (débranchement) sur les hétéropolysaccharides (arabinanes, arabinoxylanes et arabinogalactanes) pour permettre une amélioration efficace de la digestibilité des aliments par les animaux. Cette amélioration de la digestibilité des aliments pour animaux permet d'augmenter leur valeur nutritionnelle. Ainsi, les enzymes ayant une spécificité (stéréo-spécificité, énantiosélectivité), une activité ou une affinité améliorée envers les substrats naturels arabinoxylanes et arabinanes présentent un grand intérêt pour l'alimentation animale.

En outre, avant l'hydrolyse des liaisons arabinofuranosyl, il est au préalable indispensable de dépolymériser les molécules complexes telles que la cellulose. Les enzymes cellulolytiques (cellulases) de champignon possèdent en général un domaine fCDB (domaine de fixation à la cellulose de type fongique) qui joue un rôle prépondérant dans cette dépolymérisation de la cellulose.

La présente invention décrit une L-arabinofuranosidase B (ABFB-2) de *Penicillium funiculosum,* adaptée pour une application dans la nutrition animale ainsi que le gène codant pour cette enzyme. L'invention concerne aussi les homologues, les variants et les fragments de l'ABFB-2 conservant les mêmes propriétés catalytiques.

Avantageusement, les enzymes ABFB selon l'invention présentent un pH optimal très acide (pH 2.6) et conservent 55 % de leur activité à pH 1.5.

Avantageusement, l'enzyme ABFB-2 possède un domaine de fixation à la cellulose de type fongique (fCBD). Ce type de domaine a été décrit chez d'autres enzymes mais n'avait jamais été décrit pour une arabinofuranosidase fongique. La fonctionalité de ce domaine de fixation à la cellulose de l'enzyme ABFB-2 de *Penicillium funiculosum* a pu être vérifiée expérimentalement. La présence de ce domaine de fixation accroît l'affinité de l'enzyme pour son substrat et permet par conséquent d'améliorer la dégradation de la cellulose insoluble.

Ainsi, grâce à ses propriétés catalytiques et à son affinité pour la cellulose, l'ABFB-2 de *Penicillium funiculosum* est donc particulièrement adaptée à une application en alimentation animale notamment.

Les enzymes selon l'invention ont cependant également d'autres applications industrielles ou agro-industrielles. On citera notamment le traitement des jus de fruits, la fabrication du papier, la conversion de biomasses hemicellulolytiques en carburants ou produits chimiques, la préparation de boissons alcoolisées par fermentation.

### Description des séquences

SEQ ID No. 1 : Séquence génomique du gène *abfB*-2 de *Penicillium funiculosum.*
SEQ ID No. 2 : Séquence du polypeptide ABFB-2 de *Penicillium funiculosum* ayant une activité α-L-arabinofuranosidase de type B.
SEQ ID No. 3 : Domaine fCBD de l'ABFB-2 de *Penicillium funiculosum.*
SEQ ID No. 4 : Domaine de faible complexité de l'ABFB-2 de *Penicillium funiculosum.*
SEQ ID No. 5 : Domaine fCBD de la cyanomyl esterase de *Penicillium funiculosum.*
SEQ ID No. 6 : Domaine fCBD de l'endo1-4 xylanase D de *Penicillium funiculosum.*
SEQ ID No. 7 : Domaine fCBD de la xylanase cellobiohydrolase de *Penicillium funiculosum.*
SEQ ID No. 8 : Amorce PCR Xbal-abfB-2.
SEQ ID No. 9 : Amorce PCR HindIII-abfB-2.

### Description de l'invention

La présente invention concerne un polypeptide comprenant un polypeptide choisi parmi les polypeptides suivants:
- le polypeptide de la SEQ ID No. 2 ,
- le polypeptide dont la séquence est comprise entre la position 28 et la position 400 de la SEQ ID No. 2,
- un fragment du polypeptide de la SEQ ID No. 2 ayant une activité α-L-arabinofuranosidase B,
- un polypeptide ayant une activité α-L-arabinofuranosidase B et présentant au moins 80 % d'identité avec le polypeptide de la SEQ ID No. 2.

L'invention concerne aussi un polynucléotide, codant pour une activité α-L-arabinofuranosidase B, choisi parmi les polynucléotides suivants :
- le polynucléotide dont la séquence est comprise entre la position 268 et la position 1470 de la SEQ ID No. 1,
- le polynucléotide dont la séquence est comprise entre la position 349 et la position 1470 de la SEQ ID No. 1
- un polynucléotide codant pour un polypeptide selon la revendication 1.

Un autre objet de la présente invention est un polynucléotide ayant la séquence représentée à la SEQ ID No. 1 ou la séquence complémentaire à la SEQ ID No. 1.

L'invention se rapporte aussi à des cassettes d'expression comprenant dans le sens de la transcription:
- un promoteur fonctionnel dans un organisme hôte;
- un polynucléotide selon l'invention; et
- une séquence terminatrice fonctionnelle dans le même organisme hôte.

Un autre objet de l'invention est un vecteur comprenant un polynucléotide selon l'invention et/ou une cassette d'expression selon l'invention.

L'invention concerne aussi un organisme hôte transformé avec un polynucléotide selon l'invention, une cassette d'expression selon l'invention et/ou un vecteur selon l'invention.

Dans un mode de réalisation de l'invention, l'organisme hôte est choisi parmi les levures et les champignons filamenteux.

De préférence, l'organisme hôte est une souche de *Penicillium funiculosum.*

L'invention se rapporte également à un additif nutritionnel pour animaux comprenant un polypeptide selon l'invention, un organisme hôte selon l'invention ou un moût de fermentation d'un organisme hôte selon l'invention.

Préférentiellement, cet additif nutritionnel se présente sous forme liquide ou sous forme de poudre.

Un autre aspect de l'invention est un aliment pour animaux comprenant une base nutritionnelle pour animaux et un additif nutritionnel pour animaux selon l'invention.

L'invention concerne aussi l'utilisation d'un polypeptide ABFB selon l'invention ou d'un organisme hôte selon l'invention pour la fabrication d'un additif nutritionnel pour animaux ou d'un aliment pour animaux.

Un autre objet de l'invention est l'utilisation d'un polypeptide ABFB selon l'invention ou d'un organisme hôte selon l'invention pour l'hydrolyse des liaisons α-L-arabinofuranosyl des composés arabinofuranosyl-oligosaccharides.

### Polypeptides

La présente invention concerne donc des polypeptides ABFB ayant une activité α-L-arabinofuranosidase B. De préférence, ces polypetides sont isolés de *Penicillium funiculosum.*

On entend par "α-L-arabinofuranosidase B", des α-L-arabinofuranosidases (EC 3.2.1.55) de type B (GH 54) qui catalysent l'hydrolyse des liaisons α-1,5 ; α-1,3 et α-1,2 des chaînes latérales contenues dans les composés arabinofuranosyl-oligosaccharides.

Les polypeptides de la présente invention sont adaptés à l'utilisation en nutrition animale.

On entend par "polypeptide adapté à l'utilisation dans la nutrition animale", un polypeptide dont les caractéristiques sont telles qu'il convient pour la nutrition animale. Les caractéristiques essentielles à une utilisation en nutrition animale sont notamment le pH et la température auxquels l'enzyme est active. En effet, le pH du système digestif des animaux est acide et il est donc essentiel que l'enzyme reste active à ce pH, ce afin de conserver son activité dans l'hydrolyse des résidus L-arabinose. En outre, la mise en forme de l'enzyme dans un additif nutritionnel ou dans l'aliment de l'animal implique des traitements et une température supérieure à la température ambiante. L'activité des enzymes utilisées doit donc être stable dans les conditions des procédés, notamment les conditions de températures.

Selon un mode de réalisation de la présente invention, le polypeptide présente une activité α-L-arabinofuranosidase à un pH acide, par exemple inférieur à 4,5, de préférence inférieur à 4. Egalement, selon un mode de réalisation de la présente invention, le polypeptide présente une activité α-L-arabinofuranosidase optimale entre pH 1,5 et pH 3,5.

Selon un mode de réalisation préférée de la présente invention, le polypetide présente une activité α-L-arabinofuranosidase à des températures supérieures à la température ambiante. De préférence, le polypeptide de la présente invention possède une activité α-L-arabinofuranosidase optimale à une température comprise entre 30°C et 70°C, plus préférentiellement entre 40°C et 60°C.

L'α-L-arabinofuranosidase B-2 de la souche IMI378536 de *Penicillium funiculosum* est représentée à la SEQ ID No.2.

Dans un mode de réalisation préféré, les polypeptides selon l'invention sont glycosylés. Le polypeptide de la SEQ ID No. 2 possède notamment des sites de N-glycosylation à l'acide aminé 123 et à l'acide aminé 127. Dans un mode de réalisation préféré, les résidus asparagine en position 123 et 127 du polypeptide de la SEQ ID No. 2 sont glycosylés.

L'α-L-arabinofuranosidase B de *Penicillium funiculosum* est une enzyme secrétée par le champignon dans son environnement extracellulaire. Le polypeptide de la SEQ ID No. 2 comprend ainsi un peptide signal de 27 acides aminés. L'invention a également pour objet le polypeptide mature obtenu après clivage du peptide signal. En particulier, l'invention se rapporte au polypeptide dont la séquence est comprise entre la position 28 et la position 400 de la SEQ ID No. 2. Dans un autre mode de réalisation, le peptide signal du polypeptide de la SEQ ID No. 2 peut être remplacé par un peptide signal hétérologue pour l'expression et la secrétion du polypeptide de la SEQ ID No. 2 par un organisme hôte hétérologue.

L'invention se rapporte également à des fragments du polypeptide de la SEQ ID No. 2 ayant une activité α-L-arabinofuranosidase B.

Le terme "fragment" d'un polypeptide désigne un polypeptide comprenant une partie mais pas la totalité du polypeptide dont il est dérivé. L'invention concerne ainsi un polypeptide comprenant un fragment d'au moins 100, 200 ou 300 acides aminés du polypeptide de la SEQ ID No. 2.

Ce fragment du polypeptide de la SEQ ID No. 2 conserve son activité α-L-arabinofuranosidase. L'invention concerne donc les fragments biologiquement actifs du polypeptide de la SEQ ID No. 2. Le terme "fragment biologiquement actif" désigne un fragment d'un polypeptide conservant la fonction du polypeptide dont il est dérivé. Les fragments biologiquement actifs du polypeptide de la SEQ ID No. 2 conservent ainsi la fonction du polypeptide ABFB-2 de *Penicillium funiculosum.* Ces fragments biologiquement actifs ont une activité de α-L-arabinofuranosidase B. De préférence, les fragments de l'ABFB selon l'invention possèdent un domaine de fixation à la cellulose. Dans un mode de réalisation préféré, le domaine de fixation à la cellulose a la séquence décrite à la SEQ ID No. 3. Préférentiellement, ces fragments présentent une activité α-L-arabinofuranosidase optimale à pH 2.6.

Les méthodes de préparation de fragments d'un polypeptide ainsi que les techniques de mesure de l'activité α-L-arabinofuranosidase B sont bien connues de l'homme du métier.

L'invention a aussi pour objet des polypeptides ayant une activité L-arabinofuranosidase B et présentant au moins 90 % d'identité avec le polypeptide de la SEQ ID No. 2. De préférence, ces polypeptides ont les mêmes propriétés et notamment les mêmes propriétés catalytiques que les polypeptides de la SEQ ID No. 2. Préférentiellement, ces polypeptides sont isolés d'autres souches de *Penicillium funiculosum* ou d'autres champignons filamenteux. Alternativement, ces polypeptides peuvent être obtenus par des techniques de mutagenèse dirigée par exemple.

L'invention a pour objet des polypeptides présentant au moins 80 %, 90%, 95%, 98% et préférentiellement au moins 99% d'acides aminés identiques avec le polypeptide de la SEQ ID No. 2.

Par acides aminés identiques, on entend des acides aminés invariants ou inchangés entre deux séquences. Ces polypeptides peuvent présenter une délétion, une addition ou une substitution d'au moins un acide aminé par rapport au polypeptide de la SEQ ID No. 2.

L'invention a également pour objet des polypeptides présentant au moins 90%, 95%, 98% et préférentiellement au moins 99% de similarité avec le polypeptide de la SEQ ID No. 2.

Par similarité, on entend la mesure de la ressemblance entre séquences protéiques ou nucléiques. Ces polypeptides peuvent présenter une délétion, une addition ou une substitution d'au moins un acide aminé par rapport au polypeptide de la SEQ ID No. 2. Le degré de similarité entre deux séquences, quantifié par un score, est basé sur le pourcentage d'identités et/ou de substitutions conservatrices des séquences.

Les méthodes de mesure et d'identification du degré d'identité et du degré de similarité entre polypeptides sont connues de l'homme du métier. On peut employer par exemple Vector NTi 9.1.0, programme d'alignement AlignX (Clustal W algorithm) (Invitrogen INFORMAX, http://www.invitrogen.com). De préférence, on utilise les paramètres par défaut.

Les polypeptides selon l'invention sont isolés ou purifiés de leur environnement naturel. Les polypeptides peuvent être préparés au moyen de différents procédés. Ces procédés sont notamment la purification à partir de sources naturelles telles que des cellules exprimant naturellement ces polypeptides, la production de polypeptides recombinants par des cellules hôtes appropriées et leur purification ultérieure, la production par synthèse chimique ou, enfin, une combinaison de ces différentes approches. Ces différents procédés de production sont bien connus de l'homme du métier. Ainsi, les polypeptides ABFB de la présente invention peuvent être isolés à partir de *Penicillium funiculosum.* Dans un autre mode de réalisation, les polypeptides ABFB de la présente invention sont isolés à partir d'organisme hôtes recombinants exprimant un polypeptide ABFB selon l'invention.

L'invention a également pour objet des protéines de fusion, des protéines recombinantes ou des protéines chimères comprenant les polypeptides selon l'invention. Le terme "polypeptide" désigne également des protéines ainsi que des polypeptides modifiés.

Les polypeptides selon la présente invention ont une activité ABFB. De préférence, les polypeptides selon l'invention possèdent un domaine de fixation à la cellulose. Dans un mode de réalisation préféré, le domaine de fixation à la cellulose a la séquence décrite à la SEQ ID No. 3. Préférentiellement, les polypeptides présentent une activité α-L-arabinofuranosidase B optimale à pH 2.6. Préférentiellement, les polypeptides présentent une activité α-L-arabinofuranosidase B optimale à 50°C.

### Polynucléotides

L'invention concerne aussi des polynucléotides codant pour une activité α-L-arabinofuranosidase B. De préférence, ces polynucléotides codent pour une α-L-arabinofuranosidase B de *Penicillium funiculosum.*

Selon la présente invention, on entend par "polynucléotide " une chaine nucléotidique simple brin ou son complémentaire de ADN ou ARN, ou une chaine nucléotidique double brin pouvant être de type ADN complémentaire ou génomqiue. De préférence, les polynucléotides de l'invention sont de type ADN, notamment d'ADN double brin. Le terme "polynucléotide" désigne également les polynucléotides modifiés.

Les polynucléotides de la présente invention sont isolés ou purifiés de leur environnement naturel. De préférence, les polynucléotides de la présente invention peuvent être préparés par les techniques classiques de biologie moléculaire telles que décrites par Sambrook et al. (Molecular Cloning : A Laboratory Manual, 1989) ou par synthèse chimique.

Dans un premier mode de réalisation, l'invention se rapporte au polynucléotide dont la séquence est comprise entre la position 268 et la position 1470 de la SEQ ID No. 1. Ce polynucléotide code pour l'enzyme ABFB-2 de la SEQ ID No. 2 de *Penicillium funiculosum.*

Dans un deuxième mode de réalisation, l'invention concerne le polynucléotide dont la séquence est comprise entre la position 349 et la position 1470 de la SEQ ID No. 1. Ce polynucléotide code pour le polypeptide mature ABFB-2 de *Penicillium funiculosum* après clivage du peptide signal.

L'invention concerne aussi des polynucléotides présentant au moins 70 %, 75%, 80%, 85%, 90%, 95%, 98% et de préférence au moins 99% d'identité avec le polynucléotide dont la séquence est comprise entre la position 268 et la position 1470 de la SEQ ID No. 1 et/ou avec le polynucléotide dont la séquence est comprise entre la position 349 et la position 1470 de la SEQ ID No. 1. Ces polynucléotides codent pour une activité α-L-arabinofuranosidase B. De préférence, ces polynucléotides codent pour une α-L-arabinofuranosidase B de *Penicillium funiculosum*.

Par nucléotides identiques, on entend des nucléotides invariants ou inchangés entre deux séquences. Ces polynucléotides peuvent présenter une délétion, une addition ou une substitution d'au moins un nucléotide par rapport au polynucléotide de référence.

L'invention concerne aussi des polynucléotides présentant au moins 70 %, 75%, 80%, 85%, 90%, 95%, 98% et de préférence au moins 99% de similarité avec le polynucléotide dont la séquence est comprise entre la position 268 et la position 1470 de la SEQ ID No. 1 et/ou avec le polynucléotide dont la séquence est comprise entre la position 349 et la position 1470 de la SEQ ID No. 1. Ces polynucléotides codent pour une activité α-L-arabinofuranosidase B. De préférence, ces polynucléotides codent pour une α-L-arabinofuranosidase B de *Penicillium funiculosum.*

Par similarité, on entend la mesure de la ressemblance entre séquences protéiques ou nucléiques. Ces polynucléotides peuvent présenter une délétion, une addition ou une substitution d'au moins un nucléotide par rapport au polynucléotide de référence. Le degré de similarité entre deux séquences, quantifié par un score, est basé sur le pourcentage d'identités et/ou de substitution conservatrices des séquences.

Les méthodes de mesure et d'identification du degré d'identité et du degré de similarité entre les séquences d'acides nucléiques sont bien connues de l'homme du métier. On peut employer par exemple Vector NTi Vector NTi 9.1.0, programme d'alignement AlignX (Clustal W algorithm) (Invitrogen INFORMAX, http://www.invitrogen.com). De préférence, on utilise les paramètres par défaut.

Préférentiellement, les polynucléotides présentant un degré de similarité avec un polynucléotide de référence conservent la fonction de la séquence de référence. Dans le cas présent, les polynucléotides codent pour une activité α-L-arabinofuranosidase B.

L'invention concerne aussi des polynucléotides capables de s'hybrider de manière sélective avec le polynucléotide dont la séquence est comprise entre la position 268 et la position 1470 de la SEQ ID No. 1 et/ou avec le polynucléotide dont la séquence est comprise entre la position 349 et la position 1470 de la SEQ ID No. 1. De préférence, l'hybridation sélective est effectuée dans des conditions de moyenne stringence et préférentiellement dans des conditions de forte stringence. Ces polynucléotides codent pour une activité α-L-arabinofuranosidase B. De préférence, ces polynucléotides codent pour une α-L-arabinofuranosidase B de *Penicillium funiculosum*.

Par " séquence capable de s'hybrider de manière sélective ", on entend selon l'invention les séquences qui s'hybrident avec la séquence de référence à un niveau supérieur au bruit de fond de manière significative. Le niveau du signal généré par l'interaction entre la séquence capable de s'hybrider de manière sélective et les séquences de référence est généralement 10 fois, de préférence 100 fois plus intense que celui de l'interaction des autres séquences d'ADN générant le bruit de fond. Les conditions d'hybridation stringentes permettant une hybridation sélective sont bien connues de l'homme du métier. En général la température d'hybridation et de lavage est inférieure d'au moins 5°C au Tm de la séquence de référence à un pH donné et pour une force ionique donnée. Typiquement la température d'hybridation est d'au moins 30°C pour un polynucléotide de 15 à 50 nucléotides et d'au moins 60°C pour un polynucléotide de plus de 50 nucléotides. A titre d'exemple, l'hybridation est réalisée dans le tampon suivant: 6X SSC, 50 mM Tris-HCl (pH 7.5), 1 mM EDTA, 0.02% PVP, 0.02% Ficoll, 0.02% BSA, 500 µg/ml sperme de saumon denaturé DNA. Les lavages sont par exemple réalisés successivement à faible stringence dans un tampon 2X SSC, 0,1% SDS, à moyenne stringence dans un tampon 0,5X SSC, 01% SDS et à forte stringence dans un tampon 0,1X SSC, 0,1% SDS. L'hybridation peut bien entendu être effectuée selon d'autres méthodes usuelles bien connues de l'homme du métier (voir notamment Sambrook et al., Molecular Cloning : A Labratory Manual, 1989). De préférence, les polynucléotides s'hybridant de manière sélective à un polynucléotide de référence conservent la fonction de la séquence de référence. Dans le cas présent, les polynucléotides, s'hybridant de manière sélective avec le polynucléotide dont la séquence est comprise entre la position 268 et la position 1470 de la SEQ ID No. 1 et/ou avec le polynucléotide dont la séquence est comprise entre la position 349 et la position 1470 de la SEQ ID No. 1, codent pour une activité α-L-arabinofuranosidase B.

L'invention se rapporte de manière générale aux polynucléotides codant pour les polypeptides selon l'invention. En raison de la dégénérescence du code génétique, différents polynucléotides peuvent coder pour un même polypeptide.

Un autre objet de la présente invention est un polynucléotide dont la séquence est représentée à la SEQ ID No. 1. Le polynucléotide de la SEQ IDNo. 1 comprend des séquences flanquant le cadre ouvert de lecture (ORF) du gène *abfB-*2 de *Penicillium funiculosum.* Il s'agit notamment des séquences promotrices et terminatrices du gène *abfB*-2. Le gène *abfB* peut être exprimé à partir de ses séquences régulatrices homologues notamment pour une surexpression dans *Penicillium funiculosum* ou dans d'autres champignons filamenteux.

Dans un autre mode de réalisation, le gène *abfB* peut être exprimé dans différents organismes hôtes tels que les bactéries, les levures et les champignons par exemple. Le gène *abfB* peut être exprimé dans un organisme hôte sous le contrôle du promoteur de la SEQ ID No. 1 de la présente invention ou sous le contrôle d'un promoteur hétérologue.

### Cassettes d'expression

Selon un mode de réalisation de l'invention, un polynucléotide codant pour un polypeptide selon l'invention est inséré dans une cassette d'expression en utilisant des techniques de clonage bien connues de l'homme du métier. Cette cassette d'expression comprend les éléments nécessaires à la transcription et à la traduction des séquences codant pour les polypeptides selon l'invention.

Avantageusement, cette cassette d'expression comprend à la fois des éléments permettant de faire produire un polypeptide par une cellule hôte et des éléments nécessaires à la régulation de cette expression.

Ces cassettes d'expression comprennent dans le sens de la transcription:
- un promoteur fonctionnel dans un organisme hôte;
- un polynucléotide selon l'invention;
- une séquence terminatrice fonctionnelle dans le même organisme hôte.

Tout type de séquence promotrice peut être utilisée dans les cassettes d'expression selon l'invention. Le choix du promoteur dépendra notamment de l'organisme hôte choisi pour l'expression du gène d'intérêt. Certains promoteurs permettent une expression constitutive alors que d'autres promoteurs sont au contraire inductibles. Parmi les promoteurs fonctionnels dans les champignons, on citera notamment celui de glyceraldehyde-3-phosphate deshydrogenase d'*Aspergillus nidulans* (Roberts et al., Current Genet. 15:177-180, 1989). Parmi les promoteurs fonctionnels dans les bactéries, on citera notamment celui de la RNA polymérase du bacteriophage T7 (Studier et al., Methods in enzymology 185:60-89, 1990). Parmi les promoteurs fonctionnels dans les levures, on citera le promoteur du gène *GAL1* (Elledge et al., Proc Natl Acad Sciences, USA. 88:1731-1735, 1991) ou les promoteurs GAL4 et *ADH* de *S.cerevisiae.* Tous ces promoteurs sont décrits dans la littérature et bien connus de l'homme du métier.

Pour l'expression dans *Penicillium funiculosum,* on choisira par exemple des cassettes d'expression comprenant un promoteur histone H4.B, un promoteur acide aspartyl protéase ou un promoteur csl13 (WO 00/68401).

Les cassettes d'expression, selon la présente invention, peuvent en outre inclure toute autre séquence nécessaire à l'expression des polypeptides ou des polynucléotides comme par exemple des éléments de régulation ou des séquences signal permettant la sécrétion des polypeptides produits par l'organisme hôte. On peut notamment utiliser toute séquence de régulation permettant d'augmenter le niveau d'expression de la séquence codante insérée dans la cassette d'expression. Selon l'invention, on peut notamment utiliser, en association avec la séquence de régulation promotrice, d'autres séquences de régulation, qui sont situées entre le promoteur et la séquence codante, telles que des activateurs de transcription ("enhancer").

Une grande variété de séquences terminatrices sont utilisables dans les cassettes d'expression selon l'invention, ces séquences permettent la terminaison de la transcription et la polyadénylation de l'ARNm. Toute séquence terminatrice fonctionnelle dans l'organisme hôte sélectionné peut être utilisée.

Pour l'expression dans *Penicillium funiculosum*, on choisira par exemple des cassettes d'expression comprenant un terminateur histone H4.B, un terminateur acide aspartyl protease ou un terminateur csl13 (WO 00/68401).

La présente invention a également pour objet un polynucléotide comprenant une cassette d'expression selon l'invention, avantageusement les cassettes d'expression selon la présente invention sont insérées dans un vecteur.

### Vecteurs

La présente invention concerne donc également des vecteurs de réplication ou d'expression pour la transformation d'un organisme hôte comprenant au moins un polynucléotide ou une cassette d'expression selon la présente invention. Ce vecteur peut notamment correspondre à un plasmide, un cosmide, un bactériophage ou un virus dans lequel est inséré un polynucléotide ou une cassette d'expression selon l'invention. Les techniques de construction de ces vecteurs et d'insertion d'un polynucléotide de l'invention dans ces vecteurs sont bien connues de l'homme du métier. De manière générale, tout vecteur capable de se maintenir, de s'autorépliquer ou de se propager dans une cellule hôte afin d'induire notamment l'expression d'un polynucléotide ou d'un polypeptide peut être utilisé. L'homme du métier choisira les vecteurs appropriés en fonction de l'organisme hôte à transformer, et en fonction de la technique de transformation mise en oeuvre.

Les vecteurs de la présente invention sont notamment utilisés pour transformer un organisme hôte en vue de la réplication du vecteur et/ou de l'expression d'un polypeptide selon l'invention dans l'organisme hôte.

L'invention concerne aussi une méthode pour préparer un polypeptide selon l'invention comprenant les étapes suivantes:
- on transforme un organisme hôte avec un vecteur d'expression comprenant une cassette d'expression selon l'invention et/ou avec un polynucléotide selon l'invention,
- on isole les polypeptides produits par l'organisme hôte.

### Organismes hôtes

La présente invention a également pour objet, un procédé de transformation d'un organisme hôte par intégration dans le dit organisme hôte d'au moins un polynucléotide ou d'une cassette d'expression ou d'un vecteur selon l'invention. Le polynucléotide peut être intégré dans le génome de l'organisme hôte ou se repliquer de manière stable dans l'organisme hôte. Les méthodes de transformation des organismes hôtes sont bien connus de l'homme du métier et largement décrits dans la littérature.

La présente invention concerne également un organisme hôte transformé avec un polynucléotide, une cassette d'expression ou un vecteur selon l'invention. Par organisme hôte, on entend en particulier selon l'invention tout organisme mono ou pluricellulaire, inférieur ou supérieur, en particulier choisi parmi les bactéries, les levures et les les champignons. Par organisme hôte on entend un organisme non humain. De manière avantageuse, les levures sont choisies parmi *Pichia pastoris, Saccharomyces cerevisae, Yarrowia lipolytica* et *Schwanniomyces occidentalis*. Les champignons sont choisis parmi les *Aspergillus* et les *Penicilliums*, préférentiellement parmi *Penicillium funiculosum, Trichoderma reesei, Aspergillus niger, Aspergillus awamori, Aspergillus kawachii et Trichoderma koningii*. Dans un mode de réalisation préféré, l'organisme hôte est une souche de *Penicillium funiculosum* dans laquelle on exprime ou sur-exprime un polypeptide ABFB selon l'invention.

Les techniques de construction de vecteurs, de transformation d'organismes hôtes et d'expression de protéines hétérologues dans ces organismes sont largement décrites dans la littérature (Ausubel F.M. et al., "Current Protocols in Molecular Biology" Volumes 1 et 2, Greene Publishing Associates et Wiley -Interscience, 1989; T.Maniatis, E.F.Fritsch, J.Sambrook, Molecular Cloning A laboratory Handbook, 1982).

### Additifs alimentaires et aliments pour animaux

La présente invention concerne donc des additifs alimentaires apportant une activité α-L-arabinofuranosidase B. L'apport de ce type d'activité enzymatique permet d'améliorer la digestibilité de l'aliment et d'augmenter sa valeur nutritionnelle.

On entend par additif nutritionnel une substance ajoutée intentionnellement à un aliment, généralement en petites quantités, pour améliorer sa digestibilité ou ses caractéristiques nutritionnelles. Les additifs nutritionnels pour animaux peuvent par exemple contenir des vitamines, des sels minéraux, des acides aminés et des enzymes.

Typiquement, les additifs nutritionnels pour animaux comprennent un polypeptide selon l'invention, un organisme hôte selon l'invention où un moût de fermentation d'un organisme hôte selon l'invention. Ainsi, les polypeptides ayant une activité α-L-arabinofuranosidase B selon l'invention peuvent être purifiés ou isolés d'une souche de *Penicillium funiulosum* ou d'un organisme hôte recombinant pour la fabrication d'un additif nutritionnel pour animaux. Alternativement, une souche de *Penicillium funiculosum* ou un organisme hôte produisant des polypeptides AbfB peuvent être utilisés directement pour la fabrication d'un additif nutritionnel pour animaux. Dans un mode de réalisation préféré de l'invention, le surnageant de culture ou moût de fermentation d'une souche de *Penicillium funiculosum* ou d'un organisme hôte selon l'invention est utilisé pour la fabrication d'additifs nutritionnels pour animaux. Ce mode de réalisation est particulièrement avantageux lorsque les polypeptides ABFB sont sécrétés par la souche de *Penicillium funiculosum* ou l'organisme hôte. Habituellement, ce surnageant de culture est concentré ou lyophilisé pour la fabrication de l'additif nutritionnel.

Ainsi, l'invention concerne aussi un procédé de préparation d'enzyme ABFB comprenant les étapes suivantes :
a) mise en culture d'une souche de *Penicillium funiculosum* ou d'un organisme hôte transformé selon l'invention dans des conditions d'induction de l'expression des ABFB,
b) séparation du surnageant de culture comprenant l'enzyme ABFB.

Ce surnageant de culture ou moût de fermentation peut ensuite être concentré ou lyophilisé pour la formulation d'un additif alimentaire ou d'un aliment pour animaux.

Si l'organisme hôte ne secrète pas l'enzyme ABFB dans le milieu de culture, une étape supplémentaire de cassage des cellules et de purification de l'extrait cellulaire peut être nécessaire.

Les additifs nutritionnels de la présente invention comprennent une activité α-L-arabinofuranosidase B mais peuvent également comprendre d'autres substances nutritionnelles comme des vitamines, des acides aminés ou des sels minéraux.

Les additifs selon l'invention accroissent la digestibilité des aliments, contribuant ainsi à une meilleure valorisation nutritionnelle des régimes à base de céréales (blé, orge, maïs, avoine, seigle, ...) et de tourteaux oléagineux (soja, tournesol, colza, ...) notamment.

La présente invention concerne aussi les aliments pour animaux comprenant une base nutritionnelle et un additif nutritionnel selon l'invention. Ces aliments se présentent habituellement sous la forme de farines ou de granulés dans lesquels sont incorporés les additifs selon l'invention.

On entend par aliment tout ce qui peut servir à la nourriture des animaux.

Les aliments pour animaux comprennent un polypeptide selon l'invention, un organisme hôte selon l'invention où un moût de fermentation d'un organisme hôte selon l'invention.

Pour l'élevage intensif des animaux, ces aliments comprennent habituellement une base nutritionnelle et des additifs nutritionnels.

On entend par base nutritionnelle, ce qui constitue l'essentiel de la ration alimentaire de l'animal, constitué à titre d'exemple par un mélange de céréales, de protéines et de matières grasses d'origine animale et/ou végétale.

Les bases nutritionnelles pour animaux sont adaptées à l'alimentation de ces animaux et sont bien connues de l'homme du métier. Habituellement, ces bases nutritionnelles comprennent par exemple du maïs, du blé, du pois et du soja. Ces bases nutritionnelles sont adaptées aux besoins des différentes espèces animales auxquelles elles sont destinées. Ces bases nutritionnelles peuvent déjà contenir des additifs nutritionnels comme des vitamines, des sels minéraux et des acides aminés.

Dans un mode de réalisation préféré, l'invention concerne des aliments pour animaux monogastriques et notamment pour les volailles et les porcs. Les volailles comprennent notamment les poules pondeuses, les poulets de chair, les dindes et les canards. Les porcs comprennent notamment les porcs croissance et finition ainsi que les porcelets.

### Description des figures

Figure 1 : Schéma organisationnel du domaine fongique de fixation à la cellulose (fCBD).
Figure 2 : Détermination du pH optimum de l'enzyme ABFB-2 dans une série de tampon Mc Ilvaine (pH 2.2 à 8) à 40 °C en présence de 5 mM de PNPAF.
Figure 3 : Détermination de la température optimale de l'enzyme ABFB-2 à son pH optimum en présence de 5 mM de PNPAF.
Figure 4 : Détermination des constantes cinétiques *Kₘ* et *Vₘ* (1/Vi=f(1/S) pour l'ABFB-2 pour une gamme de PNPAF allant de 0,5 mM à 5 mM à pH 2.6 et 50°C.
Figure 5 : Valeurs d'expression différentielle des gènes *abfB*-1 et *abfB-2* en fonction des conditions de croissance de *P. funiculosum.*
Figure 6 : Cinétique de disparition de l'ABFB-2 dans le surnageant réactionnel.

L'adhésion de l'enzyme à la cellulose est suivie par mesure de l'activité α-L-arabinofuranosidase B résiduelle libre de l'ABFB-2.

### Exemples

### Analyse de la structure du gène ABFB-2

L'ABFB-2 présente moins de 62% d'identité avec d'autres enzymes ABFB de champignons filamenteux alors que ces enzymes sont en général assez conservées. Cette différence d'identité est expliquée par l'identification de deux régions distinctes. Dans la partie N terminale une région spécifique des arabinofuranosidases (10 aa jusqu' à 341 aa) et dans la région C terminale un domaine de fixation à la cellulose, de type fongique fCBD (fungal-type cellulose-binding domain), qui a été prédit par une analyse SMART (Simple Modular Architecture Research Tool) entre la position 367 et 400 aa. D'après la littérature ce type de domaine de fixation est généralement retrouvé dans les enzymes impliquées dans la dégradation de la cellulose telles que les endoglucanases (EC 3.2.1.4), cellobiohydrolases (EC 3.2.1.91) (exoglucanases), ou xylanases (EC 3.2.1.8). C'est la première fois qu'un domaine CBD est rapporté pour une arabinofuranosidase.

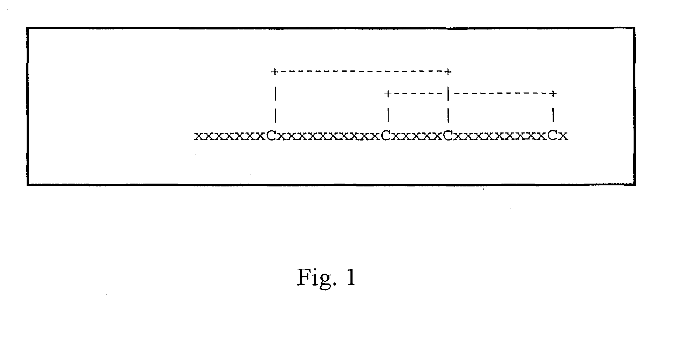

D'un point de vue structurel (séquence primaire), les cellulases et les xylanases sont constituées de deux domaines distincts, un domaine catalytique et un domaine CBD liés par une courte séquence dite de faible compléxité, riche en proline et/ou en acide aminé hydroxylés (Serine et Thréonine). Les domaines de fixation à la cellulose ont été étudiés chez un certain nombre de cellulases fongiques, et ils se caractérisent par une séquence allant jusqu' à 36 acides aminés, présente à l'extrémité N terminale (cbh-II ou egl2) ou C terminale de la protéine (cbh-I, egl1 ou egl5). De plus ce type de domaine est caractérisé par la conservation de 4 cystéines suivant la **figure 1** qui permettent la formation de ponts disulfures.

Le domaine fCBD prédit pour l'ABFB-2 de *Penicillium funiculosum* est composé de 34 acides aminés incluant les 4 cystéines conservées (THWGQ**C**GGSGYSGPTS**C**VAPYA**C**TTANPYYAQ**C**L).

Ce domaine est précédé d'une courte séquence de 23 acides aminés de faible complexité (**STSP**GSH**TTSTTLTTITSTT**AV**S**) caractéristique, riche en acides aminés portant un hydroxyle (10 thréonines et 6 sérines). Chez *Penicillium funiculosum* ce type de domaine a été décrit chez trois autres enzymes, l'endo 1-4 Xylanase, la cellobiohydrolase, et la férulique acide estérase (cyanomyl esterase). L'alignement de ces quatre domaines confirme la conservation et le positionnement des 4 cystéines au sein du fCBD.

Alignement des fCBD connus chez *Penicillium funiculosum.*

Ce type de domaine de fixation a été largement décrit chez les enzymes cellulolytiques (cellulases). L'analyse de la séquence primaire du *f*CBD par alignement blast, contre les séquences *f*CBD connues, identifie une très forte homologie avec les domaines de fixation à la cellulose de la cellobiohydrolase de type I et II, ainsi qu'avec les domaines de l'endo-glucanases de type I, II, et V issus de *Trichoderma reesei* et d'*Aspergillus niger*. Les 3 résidus tyrosines, ainsi que les résidus asparagine et glutamine, identifiés comme essentiels pour la fonctionnalité du domaine, sont conservés au sein de la séquence ABFB-2. Les études de relation structure/fonction, montrent que ce type de domaine joue un rôle prépondérant dans la dépolymérisation de la cellulose (Linder, M. & Teeri, T. T. (1997) The roles and function of cellulose-binding domains, J. Biotechnol. 57 15-28) en augmentant l'affinité de liaison de l'enzyme pour son substrat. Chez *T. reesei*, la cellobiohydrolase I (CBH I) en l'absence de son domaine *f*CBD exhibe une activité catalytique conservée, mais l'affinité de liaison de l'enzyme pour la cellulose est très fortement diminuée.

C'est la première fois qu'un domaine de fixation de ce type est identifié au sein d'une arabinofuranosidase de type B chez un champignon filamenteux. La présence de ce domaine de fixation accroît l'affinité de l'enzyme pour son substrat et permet d'améliorer par conséquent la dégradation de la cellulose insoluble.

### Mise au point du dosage de l'activité L-arabinofuranosidase B

L'activité L-arabinofuranosidase a été mesurée à partir d'une culture de *P. funiculosum* sur le milieu M2 avec un ajout mixte composé de 0,15% de Provasoy et 0,3% de cellulose au bout de 40h. Des prélèvements ont été réalisés à 48h et 72h de culture. La culture a été réalisée en erlen de 200 mL avec un volume utile de 50 mL. L'activité a été déterminée par hydrolyse de 5mM de para-nitrophényl-(-L-arabinofuranoside (PNPAF) dans un tampon 50 mM sodium acétate, pH 5. 50 µL de surnageant de culture ont été incubés avec 250µL de substrat préchauffé à 50°C pendant 15 min. La réaction a été stoppée par ajout de 500µL de 0,5 M NaOH. La libération du p-nitrophényl (PNP) est mesurée à 405 nm avec un coefficient d'extinction molaire de 17000 M-1.cm-1. Une unité enzymatique est définie comme la quantité d'enzyme qui hydrolyse 1 µmol de PNPAF par min dans les conditions décrites précédement. Pour la culture de *P. funiculosum* nous avons obtenus 20 mU.mL-1 au bout de 48h et 112 mU.mL-1 après 72h de culture. Ces résultats sont en accord avec la littérature, en effet pour *Aspergillus niger* des activités de l'ordre de 100 à 600 mU.mL-1 ont été trouvées suivant l'inducteur utilisé dans la culture.

### Clonage de l'ORF abfB-2 de P. funiculosum et transformation chez Saccharomyces cerevisiae

A partir de l'ADN génomique de *P. funiculosum,* le gène *abfB*-2 a été amplifié par PCR à l'aide d'un couple d'amorces (Hind III-abfB-2 /Xba I-abfB-2). Les conditions PCR suivantes ont été appliquées (94°C 30 sec ; 62°C 30 sec; 1 min 30 sec à 72°C) pendant 30 cycles. Le produit PCR de 1215 pb a été cloné dans un vecteur commercial pGEM-T(tm) easy.

Séquence du couple d'amorce PCR
Xba I- abfB-2 : > 5'-TCTAGAATGACGTCCAAACATAGTT-3'<
Hind III- abfB-2: > 5'-AAGCTTCTAGAGACATTGAGCGTA- 3'<

Le fragment Hind III/Xba I de 1215 bp a été excisé du vecteur pGEM-T et sous cloné aux sites Hind IIIIXba I dans un vecteur navette (plac195-PGK/CYC1). Pour l'expression hétérologue, le gène *abfB*-2 se trouve donc sous la dépendance du promoteur constitutif PGK du gène codant pour la phospho-glycérate kinase *(S. cerevisiae)* et du terminateur *CYC1 (S. cerevisiae)* du gène codant pour une activité cytochrome C oxydase. Le nouveau vecteur d'expression est dénommé pOT-02.

La souche *S.cerevisiae* JF #1194 (CEN.PK113-5D), clone issu de la souche CEN.PK 122 portant l'auxotrophie *ura* 3-52, à été transformée (méthode au lithium acétate/ choc thermique) par le vecteur d'expression pOT-02. Les souches transformantes ont été sélectionnées par complémentation phénotypique sur boites sélectives sans uracile (marqueur *URA3*).

Six transformants ont été sélectionnés pour tester la présence d'une activité arabinofuranosidase B dans le surnageant de culture. Les transformants ont été cultivés dans 50 mL de milieu minimum sans uracile (hormis la souche contrôle sauvage) pendant 24 heures. L'activité arabinofuranosidase a été dosée sur les surnageants de culture à l'aide de la méthode décrite dans le paragraphe précédent.

### Détermination du pH optimum

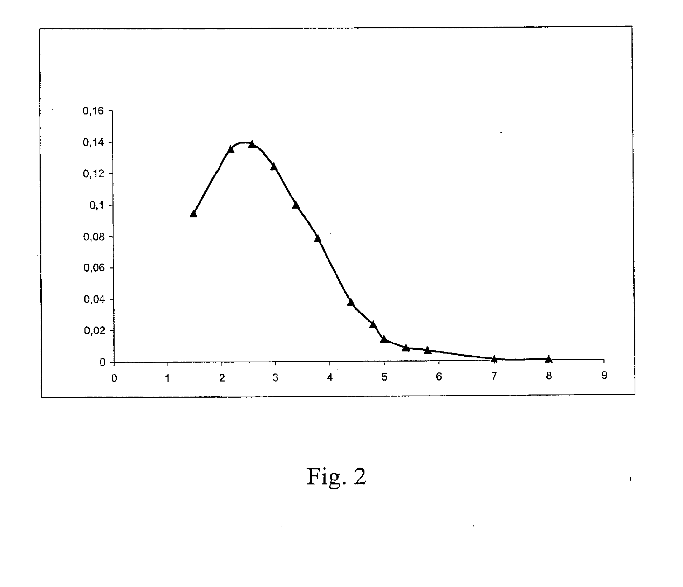

Le gène *abfB-2* codant pour une activité arabinofuranosidase B issue de *P. funiculosum* a été cloné chez *S*. *cerevisiae.* Après vérification de la présence d'une activité arabinofuranosidase B chez plusieurs transformants, un transformant a été choisi et l'activité ABFB à été dosée sur le surnageant de culture après 24 h de croissance. Les cultures ont été réalisées en erlen de 200 mL (Vu 50 mL). L'activité a été déterminée en présence de 5 mM de p-nitrophényl-α-L-arabinofuranoside (PNPAF) dans une série de tampon Mc Ilvaine (pH 2,2 à 8,0). 80 µL de surnageant de culture ont été incubés avec 320 µL de substrat préchauffé à 40°C pendant 10 min. La réaction a été stoppée par ajout de 1 mL de Na₂CO₃ 1 M. La libération du p-nitrophényl est mesurée à 405 nm⁻¹. Une unité enzymatique est définie comme la quantité d'enzyme qui hydrolyse 1 µmol de PNPAF par min. La courbe d'activité est représentée à la **figure 2**. L'ABFB-2 a un optimum d'activité à pH 2.6 et conserve 55% de son activité à pH 3,8. C'est la première fois qu'un pH optimum si bas est observé pour une arabinofuranosidase, et qu'elle présente 68% d'activité à pH 1,5 dans une solution 50 mM HCl.

### Détermination de la température optimale

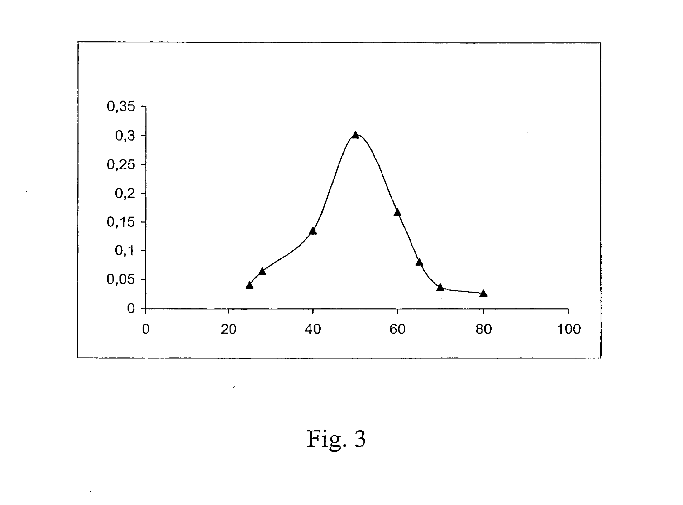

Selon le même protocole, nous avons déterminé la température optimale d'activité de l'ABFB-2. L'enzyme a été incubée 10 min à chacune des températures dans un tampon Mc Ilvaine au pH 2,6. La courbe d'activité est présentée à la **figure 3**.

La littérature décrit une gamme de température optimale des ABFB comprise en 40 et 60°C. L'ABFB-2 de *P. funiculosum* présente un optimum d'activité à 50°C. En se plaçant aux pH et aux températures optimales de l'enzyme (pH 2,6 et 50°C), nous remarquons que l'activité pour l'ABFB-2 est 20 fois supérieure à l'activité déterminée dans un tampon acétate pH 5 et 40°C (305 mU vs 15 mU).

### Détermination de Kₘ et Vₘ

Les constantes cinétiques (*Kₘ* et *Vₘ*) de l'ABFB-2 chacune des deux ont été déterminées par mesure de l'hydrolyse du PNPAF au cours du temps, dans les conditions optimales déterminées précédemment.

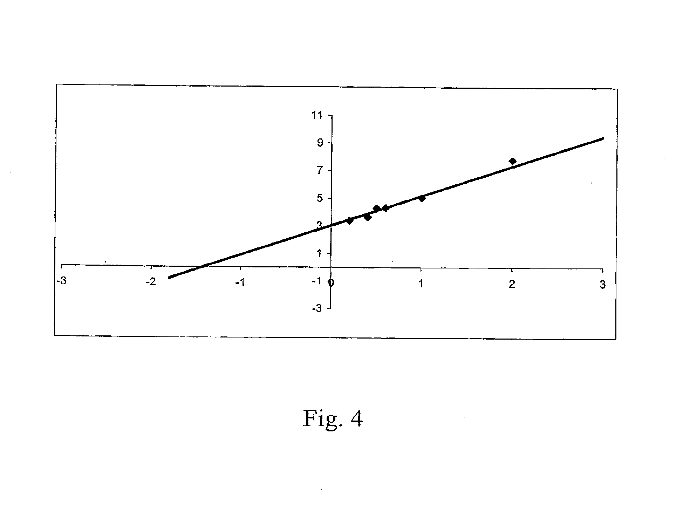

Les gammes de concentration en substrat (PNPAF) ont été établies entre 0,5 et 5 mM dans un tampon à pH 2,6. La cinétique d'hydrolyse a été suivie pendant 10 minutes à 50°C. Afin d'obtenir les constantes cinétiques de chacune des enzymes les résultats ont été traités selon la méthode des doubles inverses (Lineweark et Burk) et sont présentés dans la **figure 4****.**

Les constantes cinétiques ont été déterminées par hydrolyse du PNPAF dans des conditions optimales. La valeur de Km est de 0,7 mM pour l'ABFB-2. Par comparaison dans la littérature les valeurs de Km pour ce type d'enzyme varie de 0,05 à 1,2 mM selon le genre et l'espèce fongique étudié. L'ABFB-2 présente une vitesse d'hydrolyse maximale (*Vₘ*) de 328 mol PNPAF/mole d'enzyme/min, dans les conditions décrites précédement.

### Détermination du poids moléculaire de l'enzyme ABFB-2

Afin de déterminer le poids moléculaire de l'enzyme ABFB-2, le surnageant de culture, issu de la croissance en milieu minimum du mutant (*S.cerevisae*), a été concentré 200 fois, dénaturé par ébullition à 100°C pendant 5 min, puis déposé dans un gel de polyacrylamide-SDS.

On observe que la quantité de protéines extracellulaires est extrêmement faible dans la souche sauvage. Pour les mutants, l'enzyme ABFB-2 est sécrétée dans le surnageant de culture. Elle est majoritaire par rapport au niveau basal des protéines extracellulaires de *S.cerevisiae*. Pour l'enzyme ABFB-2, la bande électrophorétique obtenue est diffuse ce qui suggère une forte glycosylation de l'enzyme.

La détermination du poids moléculaire a été effectuée à l'aide du marqueur de taille SeeBlue (Invitrogen). Les résultats sont présentés dans le tableau 1.

**Tableau 1 : Poids Moléculaire ABFB-2 en KDa**

| | **PM prédit** | **PM estimé sur gel** |
|---|---|---|
| **ABFB-2** | **41** | **55** |

Nous avons comparé le poids moléculaire prédit par l'algorithme de Vector NTi et le poids obtenu par migration électrophorétique dans un gel dénaturant SDS-PAGE. Nous observons une sur estimation du poids moléculaire de l'enzyme dans le gel SDS-PAGE. Une forte glycosylation de l'enzyme est en effet suggérée par la visualisation sur gel d'une bande électrophorétique diffuse (O et N glycosilations). Ces glycosylations surviennent lors de la maturation des protéines dans l'organisme d'expression.

### Analyse du profil d'expression du gène abfB-2 chez Penicillium funiculosum

*Penicillium funiculosum* possède deux gènes codant pour des α-L-arabinofuranosidase B : les gènes *abfB*-1 et *abfB-2.* Les profils d'expression de ces gènes dans différentes conditions de culture de P.funiculosum ont été comparés.

*P. funiculosum* a été cultivé dans des conditions d'induction d'enzymes celluloytiques et hémi-cellulolytiques (milieu de croissance industriel de type M2) et dans des conditions de non production (milieu minimum glucose MO). Après 40h de croissance les cultures ont été arrêtées, le mycélium a été récupéré, et les ARN totaux ont été extraits. La quantité et la qualité des ARN ont été appréciées par mesure de l'absorbance à 260nm et à 280 nm (Ratio 260/280, > 1,8). Le niveau de transcrits codant pour les activités arabinofuranosidases de type B (ABFB-1 et ABFB-2) ont été quantifiés dans chacune des deux conditions (MO et M2) par PCR quantitative en temps réel.

Le gène codant pour la tubuline (*tub*-1) de *P. funiculosum* a été utilisé comme contrôle dans les deux conditions. Ce gène code pour une protéine de structure indispensable pour l'intégrité de la cellule. Ce gène est utilisé de façon courante comme gène de référence, car il présente un niveau d'expression constant quelque soit la condition de culture utilisée (ubiquitaire).

Des amorces spécifiques pour la PCR quantitative ont été dessinées pour chacun des gènes (*abfB*-1, *abfB*-2, et *tub*-1). Pour les deux conditions de croissance (M0 et M2), 2 µg d'ARN totaux ont été rétro-transcripts. Une série de dilution des ADN complémentaires issus de la rétro-transcription a été réalisée afin de déterminer les conditions optimales d'amplification des gènes cibles (contraintes de la méthode de PCR quantitative et pour l'efficacité des ces couples d'amorces).

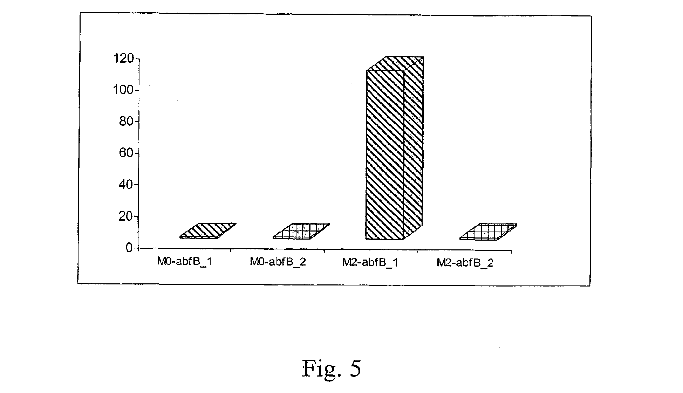

Les résultats normalisés sont présentés dans le tableau 2 et la **figure 5**.

**Tableau 2 : Valeurs d'expression différentielle des gènes abfB-1 et abfB-2 en fonction des conditions de croissance de P. funiculosum**

| | **M0** | **M2** |
|---|---|---|
| **abfb_1** | 1 | 107 |
| **abfb_2** | 1 | 1,27 |

Les régulations transcriptionnelles des gènes codant pour des activités cellulolytiques et hémi-cellulolytiques ont été décrites. L'expression de ces gènes est fortement soumise à la nature et/ou à la complexité de la source de carbone et d'azote sur laquelle le microorganisme est cultivé. Il a été rapporté une forte répression transcriptionnelle de ces gènes en présence de glucose. Cette régulation est effectuée par l'intermédiaire d'une protéine de répression catabolique CreA qui se fixe spécifiquement sur le promoteur de ces gènes et bloque leur transcription. Dans notre expérience de quantification par PCR des messagers *abfB-1* et *abfB*-2, que le niveau d'expression de ces 2 gènes dans la condition glucose (M0) est très faible. Ceci est en accord avec la littérature puisqu'il a été montré que ces gènes présentent un niveau basal d'expression même dans des conditions non favorables (absence de substrats cellulolytiques et / ou hémi cellulolytiques). Les résultats obtenus pour la condition M0 sont en accord avec la littérature. Contrairement au gène *abfB*-1*,* l'expression du gène abfB-2 n'est pas induite dans les conditions industrielles. Ceci suggère que les cocktails enzymatiques produits par *Penicillium funiculosum* pourraient être améliorés en obtenant l'expression de l'ABFB-2 par le champignon en conditions industrielles ou encore en ajoutant de l'ABFB-2 exogène au cocktail enzymatique produit.

### Détermination de la fonctionnalité du fCBD

Pour vérifier la fonctionnalité du domaine *f*CBD, des essais de fixation de l'enzyme ABFB-2 ont été réalisés sur de la cellulose microcristalline.

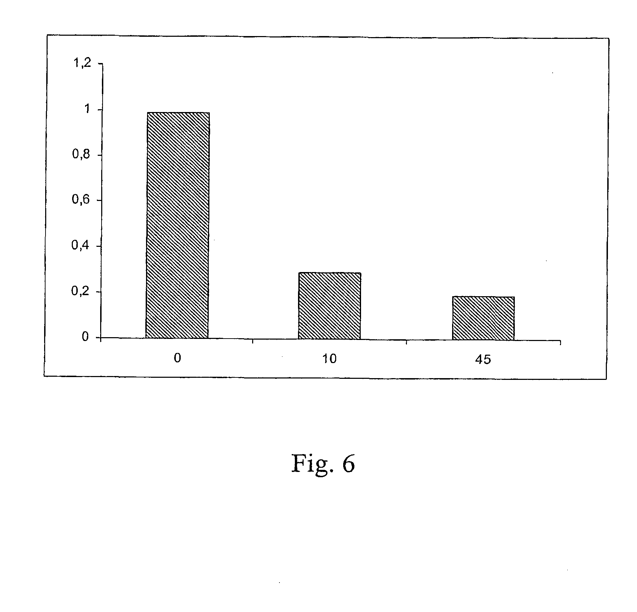

L'enzyme a été mélangée volume à volume avec 0,5% de cellulose microcristalline dans un tampon 100 mM Tris-HCl, pH 7,5 à 4°C. L'adhésion de la protéine a été suivie par mesure de l'activité arabinofuranosidase B résiduelle en présence de PNPAF dans les conditions d'hydrolyse optimales déterminées. Après différents temps de contact entre l'enzyme et la cellulose, le mélange est centrifugé et la diminution de la concentration en protéine ABFB-2 est suivie par mesure de l'activité arabinofuranosidase B dans la surnageant. Les tests de fixation ont été effectués en présence d'une réaction contrôle contenant l'enzyme ABFB-2, dans les même conditions expérimentales mais en absence de cellulose microcristalline. Un deuxième contrôle à été effectué en incubant l'enzyme ABFB-1 dans les même conditions réactionnelles en présence de cellulose microcristalline. Les résultats obtenus sont présentés dans la **figure 6**.

Nous constatons donc que le domaine de fixation à la cellulose de type fongique (fCDB) de l'enzyme ABFB-2 issu de *P. funiculosum* est fonctionnel. L'adhésion de l'enzyme à été suivie après 10 et 45 minutes de mise en contact. Après 10 min d'incubation, 70 % de la quantité totale de l'enzyme présente à l'instant initiale a adhéré à la cellulose microcristalline. Au bout de 45 minutes, 80 % de la totalité de l'enzyme présente dans la réaction à la cellulose a adhéré. Cette donnée laisse penser que l'équilibre de la réaction de contact a été atteint très rapidement, et que la cinétique d'adhésion de l'enzyme suit très certainement une loi cinétique d'ordre 1 par rapport au substrat dans les 10 premières minutes.

## Revendications

1. Polypeptide **caractérisé en ce qu'**il comprend un polypeptide choisi parmi les polypeptides suivants:
- le polypeptide de la SEQ ID No. 2,
- le polypeptide dont la séquence est comprise entre la position 28 et la position 400 de la SEQ ID No. 2,
- un fragment du polypeptide de la SEQ ID No. 2 ayant une activité α-L-arabinofuranosidase B,
- un polypeptide ayant une activité α-L-arabinofuranosidase B et présentant au moins 80 % d'identité avec le polypeptide de la SEQ ID No. 2.

2. Polynucléotide codant pour une activité α-L-arabinofuranosidase B **caractérisé en ce qu'**il est choisi parmi les polynucléotides suivants :
- le polynucléotide dont la séquence est comprise entre la position 268 et la position 1470 de la SEQ ID No. 1,
- le polynucléotide dont la séquence est comprise entre la position 349 et la position 1470 de la SEQ ID No. 1
- un polynucléotide codant pour un polypeptide selon la revendication 1.

3. Polynucléotide **caractérisé en ce qu'**il a la séquence de la SEQ ID No. 1 ou la séquence complémentaire à la SEQ ID No. 1.

4. Cassette d'expression **caractérisée en ce qu'**elle comprend dans le sens de la transcription:
- un promoteur fonctionnel dans un organisme hôte;
- un polynucléotide selon la revendication 2; et
- une séquence terminatrice dans le même organisme hôte.

5. Vecteur comprenant un polynucléotide selon l'une des revendications 2-3 et/ou une cassette d'expression selon la revendication 4.

6. Organisme hôte non humain transformé avec un polynucléotide selon l'une des revendications 2-3, une cassette d'expression selon la revendication 4 et/ou un vecteur selon la revendication 5.

7. Organisme hôte selon la revendication 6 **caractérisé en ce que** l'organisme hôte est choisi parmi les levures et les champignons filamenteux.

8. Organisme hôte selon la revendication 7 **caractérisé en ce qu'**il s'agit d'une souche de *Penicillium funiculosum.*

9. Additif nutritionnel pour animaux **caractérisé en ce qu'**il comprend un polypeptide selon la revendication 1.

10. Additif nutritionnel pour animaux **caractérisé en ce qu'**il comprend un organisme hôte selon l'une des revendications 6-8 et/ou un moût de fermentation d'un organisme hôte selon l'une des revendications 6-8.

11. Additif nutritionnel pour animaux selon l'une des revendications 9-10 **caractérisé en ce qu'**il se présente sous forme liquide ou sous forme de poudre.

12. Aliment pour animaux **caractérisé en ce qu'**il comprend une base nutritionnelle pour animaux et un additif nutritionnel pour animaux selon l'une des revendications 9-11.

13. Utilisation d'un polypeptide selon la revendication 1 ou d'un organisme hôte selon l'une des revendications 6-8 pour la fabrication d'un additif nutritionnel pour animaux ou d'un aliment pour animaux.

14. Utilisation d'un polypeptide selon la revendication 1 ou d'un organisme hôte selon l'une des revendications 6-8 pour l'hydrolyse des liaisons α-L-arabinofuranosyl des composés arabinofuranosyl-oligosaccharides.

## Patentansprüche

1. Polypeptid, **dadurch gekennzeichnet, dass** es ein Polypeptid umfasst, das aus den folgenden Polypeptiden ausgewählt ist:
- dem Polypeptid der SEQ ID Nr. 2,
- dem Polypeptid, dessen Sequenz zwischen der Position 28 und der Position 400 der SEQ ID Nr. 2 liegt,
- einem Fragment des Polypeptids der SEQ ID Nr. 2, das eine α-L-Arabinofuranosidase-B-Aktivität hat,
- einem Polypeptid, das eine α-L-Arabinofuranosidase-B-Aktivität hat und mindestens 80 % Identität mit dem Polypeptid der SEQ ID Nr.2 aufweist.

2. Polynukleotid, das für eine α-L-Arabinofuranosidase-B-Aktivität kodiert, **dadurch gekennzeichnet, dass** es aus den folgenden Polynukleotiden ausgewählt ist:
- dem Polynukleotid, dessen Sequenz zwischen der Position 268 und der Position 1470 der SEQ ID Nr. 1 liegt,
- dem Polynukleotid, dessen Sequenz zwischen der Position 349 und der Position 1470 der SEQ ID Nr. 1 liegt,
- einem Polynukleotid, das für ein Polypeptid nach Anspruch 1 kodiert.

3. Polynukleotid, **dadurch gekennzeichnet, dass** es die Sequenz der SEQ ID Nr. 1 oder die zu der SEQ ID Nr. 1 komplementäre Sequenz hat.

4. Expressionskassette, **dadurch gekennzeichnet, dass** sie in der Richtung der Transkription Folgendes umfasst:
- einen Promoter, der in einem Wirtsorganismus funktionell ist;
- ein Polynukleotid nach Anspruch 2 und
- eine Terminationssequenz in demselben Wirtsorganismus.

5. Vektor, der ein Polynukleotid nach einem der Ansprüche 2 - 3 und/oder eine Expressionskassette nach Anspruch 4 umfasst.

6. Nichtmenschlicher Wirtsorganismus, der mit einem Polynukleotid nach einem der Ansprüche 2 - 3, einer Expressionskassette nach Anspruch 4 und/oder einem Vektor nach Anspruch 5 transformiert wird.

7. Wirtsorganismus nach Anspruch 6, **dadurch gekennzeichnet, dass** der Wirtsorganismus aus Hefen und Fadenpilzen ausgewählt ist.

8. Wirtsorganismus nach Anspruch 7, **dadurch gekennzeichnet, dass** es sich um einen Stamm von *Penicillium funiculosum* handelt.

9. Ernährungsphysiologischer Zusatzstoff für Tiere, **dadurch gekennzeichnet, dass** er ein Polypeptid nach Anspruch 1 umfasst.

10. Ernährungsphysiologischer Zusatzstoff für Tiere, **dadurch gekennzeichnet, dass** er einen Wirtsorganismus nach einem der Ansprüche 6 - 8 und/oder eine Fermentationsmaische eines Wirtsorganismus nach einem der Ansprüche 6 - 8 umfasst.

11. Ernährungsphysiologischer Zusatzstoff für Tiere nach einem der Ansprüche 9 - 10, **dadurch gekennzeichnet, dass** er in flüssiger Form oder in Pulverform vorliegt.

12. Futtermittel für Tiere, **dadurch gekennzeichnet, dass** es eine ernährungsphysiologische Grundlage für Tiere und einen ernährungsphysiologischen Zusatzstoff für Tiere nach einem der Ansprüche 9 - 11 umfasst.

13. Verwendung eines Polypeptids nach Anspruch 1 oder eines Wirtsorganismus nach einem der Ansprüche 6 - 8 zur Herstellung eines ernährungsphysiologischen Zusatzstoffes für Tiere oder eines Futtermittels für Tiere.

14. Verwendung eines Polypeptids nach Anspruch 1 oder eines Wirtsorganismus nach einem der Ansprüche 6 - 8 zur Hydrolyse von α-L-Arabinofuranosyl-Bindungen von Arabinofuranosyl-Oligosaccharid-Verbindungen.

## Claims

1. Polypeptide, **characterized in that** it comprises a polypeptide chosen from the following polypeptides:
- the polypeptide of SEQ ID No. 2,
- the polypeptide whose sequence is between position 28 and position 400 of SEQ ID No. 2,
- a fragment of the polypeptide of SEQ ID No. 2 having an α-L-arabinofuranosidase B activity,
- a polypeptide having an α-L-arabinofuranosidase B activity and exhibiting at least 80% identity with the polypeptide of SEQ ID No. 2.

2. Polynucleotide encoding an α-L-arabinofuranosidase B activity, **characterized in that** it is chosen from the following polynucleotides:
- the polynucleotide whose sequence is between position 268 and position 1470 of SEQ ID No. 1,
- the polynucleotide whose sequence is between position 349 and position 1470 of SEQ ID No. 1,
- a polynucleotide encoding a polypeptide according to Claim 1.

3. Polynucleotide, **characterized in that** it has the sequence of SEQ ID No. 1 or the sequence complementary to SEQ ID No. 1.

4. Expression cassette, **characterized in that** it comprises, in the direction of transcription:
- a promoter that is functional in a host organism;
- a polynucleotide according to Claim 2; and
- a terminator sequence in the same host organism.

5. Vector comprising a polynucleotide according to one of Claims 2-3 and/or an expression cassette according to Claim 4.

6. Non-human host organism transformed with a polynucleotide according to one of Claims 2-3, an expression cassette according to Claim 4 and/or a vector according to Claim 5.

7. Host organism according to Claim 6, **characterized in that** the host organism is chosen from yeasts and filamentous fungi.

8. Host organism according to Claim 7, **characterized in that** it is a *Penicillium funiculosum* strain.

9. Nutritional additive for animals, **characterized in that** it comprises a polypeptide according to Claim 1.

10. Nutritional additive for animals, **characterized in that** it comprises a host organism according to one of Claims 6-8 and/or a fermentation broth of a host organism according to one of Claims 6-8.

11. Nutritional additive for animals according to one of Claims 9-10, **characterized in that** it is in liquid form or in powdered form.

12. Feedingstuff, **characterized in that** it comprises a nutritional base for animals and a nutritional additive for animals according to one of Claims 9-11.

13. Use of a polypeptide according to Claim 1 or of a host organism according to one of Claims 6-8 for the manufacture of a nutritional additive for animals or of a feedingstuff.

14. Use of a polypeptide according to Claim 1 or of a host organism according to one of Claims 6-8, for hydrolysing the α-L-arabinofuranosyl bonds of arabinofuranosyl-oligosaccharide compounds.
